(19) Europäisches Patentamt
European Patent Office
Office européen des brevets

(11) Veröffentlichungsnummer: **0 292 889 B1**

# (12) EUROPÄISCHE PATENTSCHRIFT

(45) Veröffentlichungstag der Patentschrift: **02.09.92**

(51) Int. Cl.[5]: **C07C 69/24**, C07C 69/96, C25B 3/02, A61K 7/46

(21) Anmeldenummer: **88108084.0**

(22) Anmeldetag: **20.05.88**

## (54) Neue Benzaldehydderivate, ihre Herstellung und Verwendung.

(30) Priorität: **29.05.87 DE 3718201**

(43) Veröffentlichungstag der Anmeldung:
**30.11.88 Patentblatt 88/48**

(45) Bekanntmachung des Hinweises auf die Patenterteilung:
**02.09.92 Patentblatt 92/36**

(84) Benannte Vertragsstaaten:
**BE CH DE FR GB IT LI NL**

(56) Entgegenhaltungen:

**THE JOURNAL OF ORGANIC CHEMISTRY, Band 50, 1985, Seiten 539-541, The American Society, Columbus, Ohio, US**

**JOURNAL OF CHEMICAL SOCIETY PERKIN I, 1978, Seiten 708-715, The Chemical Society, London, GB; A. NILSSON et al.: "Anodic Functionalisation in Synthesis. Part 1."**

(73) Patentinhaber: **BASF Aktiengesellschaft**
**Carl-Bosch-Strasse 38**
**W-6700 Ludwigshafen(DE)**

(72) Erfinder: **Grosse Brinkhaus, Karl-Heinz, Dr.**
**Kaiserslauterer Strasse 112**
**W-6702 Bad Duerkheim(DE)**
Erfinder: **Paul, Axel, Dr.**
**Kaefertaler Strasse 38**
**W-6800 Mannheim 1(DE)**
Erfinder: **Lanzendoerfer, Franz, Dr.**
**Bruchstrasse 40**
**W-6701 Ellerstadt(DE)**

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).



Rank Xerox (UK) Business Services

## Beschreibung

Die vorliegende Erfindung betrifft neue Benzaldehydderivate, ihre Herstellung und Verwendung als Riechstoffe bzw. Riechstoffvorprodukte.

Acetale von Benzaldehyden finden aufgrund ihrer im Vergleich zu den Aldehyden im alkalischen Medium größeren Stabilität breite Anwendung in der Parfümerie, besonders zur Parfümierung technischer Produkte. Auch dienen diese Acetale als Vorprodukte für Aldehyde und stellen so wertvolle Zwischenprodukte für die Synthese von Pharmaka und Lichtschutzmitteln dar.

Es wurde nun gefunden, daß die neuen Benzaldehydderivate der allgemeinen Formel

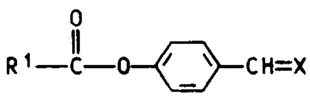

I,

in der X ein Sauerstoffatom oder zwei $R^2O$-Gruppen, in denen $R^2$ für eine Methyl- oder Ethylgruppe steht, bedeutet, $R^1$ für eine iso-Propyl- oder tert.-Butylgruppe steht und $R^1$ für den Fall, daß X für die beiden $R^2O$-Gruppen steht, auch eine Alkoxigruppe mit 1 bis 4 C-Atomen bedeuten kann, wertvolle Riechstoffe und Vorprodukte z.B. für Riechstoffe, Pharmaka und Lichtschutzmittel darstellen.

Die neuen Benzaldehydderivate dieser Erfindung sind z.B. Benzaldehyddialkylacetale der allgemeinen Formel

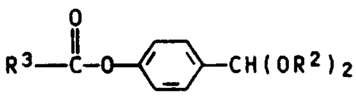

II,

oder Benzaldehyde der allgemeinen Formel

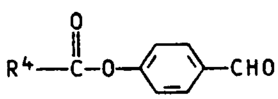

III,

in denen $R^2$ die obengenannte Bedeutung hat, $R^3$ eine iso-Propyl- oder tert.-Butylgruppe oder eine Alkoxigruppe mit 1 bis 4 C-Atomen bedeutet, und $R^4$ für eine iso-Propyl- oder tert.-Butylgruppe steht. Die Benzaldehyde der Formel III zeichnen sich durch ein süßlich-vanilleartiges Aroma aus.

In der EP 25 883 wird die Herstellung und Verwendung von 4-tert. Butoxibenzaldehyddialkylacetalen beschrieben. Der hieraus zugängliche 4-tert.-Butoxibenzaldehyd spaltet im sauren Medium die O-Schutzgruppe ab. Im Gegensatz dazu haben die erfindungsgemäßen Benzaldehyddialkylacetale der Formel II den Vorteil, daß sie als Vorstufen für substituierte Benzaldehyde herangezogen werden können, deren O-Schutzgruppe im sauren Medium nicht abspaltbar ist. Somit kommt den neuen Benzaldehyddialkylacetalen der Formel II auch eine besondere Bedeutung als Zwischenprodukte, z.B. für die Synthese von Pharmaka und Lichtschutzmitteln zu.

Aus J. Chem Soc. Perkin I, 1978, Seite 708 und der DE-PS 2848 397 ist bekannt, daß man Toluole durch anodische Oxidation in Gegenwart von Methanol und einem Leitsalz selektiv in die entsprechenden Benzaldehyddimethylacetaleüberführen kann. In J. Org. Chem. 50 (1985), 539 wird die Darstellung von p-Acetoxybenzaldehyd bzw. p-Acetoxybenzaldehyddimethylacetal durch anodische Oxidation von p-Acetoxytoluol in Gegenwart eines Leitsalzes und einer niederen aliphatischen Carbonsäure in Methanol beschrieben. Dieses Verfahren läßt sich im technischen Maßstab nur mit sehr schlechten Ausbeuten durchführen.

Es wurde nun gefunden, daß man die neuen Benzaldehyddialkylacetale der Formel II besonders vorteilhaft dadurch herstellen kann, daß man Toluole der allgemeinen Formel

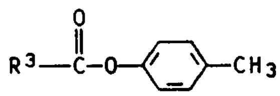 IV,

in Gegenwart eines Alkanols der Formel $R^2OH$ elektrochemisch oxidiert, wobei $R^2$ und $R^3$ die obengenannte Bedeutung haben. Nach diesem Verfahren erhält man die Benzaldehyddialkylacetale der Formel II in guten Ausbeuten.

Das erfindungsgemäße Verfahren benötigt keine besondere Elektrolysezelle. Zweckmäßigerweise verwendet man eine ungeteilte Durchflußzelle. Als Anoden können alle Anodenmaterialien, die unter den Elektrolysebedingungen stabil sind, eingesetzt werden. Bevorzugt verwendet man Graphit. Als Kathodenmaterialien sind u.a. Graphit, Eisen, Stahl, Nickel oder auch Edelmetalle geeignet. Die Elektrooxidation der Toluolderivate der Formel IV wird in Gegenwart eines Alkanols der Formel $R^2OH$ und zweckmäßigerweise in Gegenwart eines Leitsalzes durchgeführt. Der bei der Elektrooxidation eingesetzte Elektrolyt kann auch z.B. bis zu einem Equivalent, bezogen auf das Toluolderivat, einer aliphatischen Carbonsäure enthalten. Der Elektrolyt hat beispielsweise folgende Zusammensetzung:
1 bis 50 Gew.-% Toluolderivat der Formel IV
0,1 bis 4 Gew.-% Leitsalz
0 bis 8 Gew.-% einer aliphatischen Carbonsäure
50 bis 98 Gew.-% Methanol oder Ethanol
Als Leitsalze kommen die in der organischen Elektrochemie üblichen Leitsalze, wie Salze der Tetrafluoroborsäure, Salze von Alkyl- oder Arylsulfonsäuren oder Salze von Alkylschwefelsäuren sowie Salze der Perchlorsäure in Betracht. Als aliphatische Carbonsäuren sind z.B. Essigsäure oder solche Carbonsäuren geeignet, die der Formel $R^1$-COOH entsprechen, wie iso-Buttersäure und Pivalinsäure.

Man elektrolysiert bei Stromdichten von 0,5 bis 6 A/dm$^2$, bevorzugt bei 1 bis 3 A/dm$^2$. Die Elektrolysetemperaturen sind nach oben hin durch den Siedepunkt des Alkohols begrenzt. Zweckmäßigerweise elektrolysiert man bei Temperaturen von Raumtemperatur bis etwa 10 ° C unterhalb des Siedepunktes des Elektrolyten.

Die Aufarbeitung der Elektrolyseausträge nimmt man nach an sich bekannten Methoden vor. Zweckmäßigerweise wird der Elektrolyseaustrag destillativ aufgearbeitet. Überschüssiger Alkohol und die eingesetzte Carbonsäure werden zunächst abdestilliert, das Leitsalz und im Falle der Neutralisation des Elektrolyseaustrages auch das Salz der zugesetzten Carbonsäure abfiltriert und die Benzaldehyddialkylacetalderivate reindestilliert. Der Alkohol, das Leitsalz und gegebenenfalls auch die Carbonsäure können zur Elektrolyse zurückgeführt werden.

Die neuen Benzaldehyde der Formel III kann man aus den Benzaldehyddialkylacetalen der Formel II auf an sich bekannte Weise durch Hydrolyse herstellen. Dabei verfährt man z.B. so, daß man die Dialkylacetale bei Raumtemperatur mit wäßriger Mineralsäure oder wäßriger Carbonsäure versetzt und die Aldehyde nach Extraktion reindestilliert.

Beispiel 1

Elektrosynthese von 4-(Dimethoximethyl)phenylisobutyrat

Es wurde in einer ungeteilten Zelle mit 15 bipolaren Graphitelektroden elektrolysiert. Der Elektrolyt hatte die folgende Zusammensetzung:
57 g (5,0 Gew.-%) 4-Methylphenylisobutyrat
28,2 g (2,5 Gew.-%) Isobuttersäure
3,4 g (0,3 Gew.-%) Kaliumbenzosulfonat
1050 g (92,2 Gew.-%) Methanol

| | |
|---|---|
| Temperatur: | 40 ° C |
| Stromdichte: | 1 A/dm$^2$ |
| Durchfluß: | 190 l/h |

Elektrolyse mit 14,5 F/Mol 4-Methylphenylisobutyrat

Aufarbeitung:
Nach Beendigung der Elektrolyse wurde der Austrag mit $Na_2CO_3$ neutralisiert, das ausgefallene Salz abfiltriert und das Filtrat im Rotationsverdampfer eingedampft. Der Rückstand wurde in Methyl-tert.-

butylether aufgenommen und filtriert. Nach Destillation des Lösungsmittels erhielt man durch fraktionierte Destillation bei 117 °C (0,1 mbar) 40,6 g (53 %) 4-(Dimethoximethyl)phenylisobutyrat.
MS (CI): m/e = 238
$^1$H-NMR ($CDCl_3$, 200 MHz): = δ 7,40 - 7,50 (2H), 7,05 - 7,15 (2H), 5.42 (s,1H)3,32 (s,6H), 2,7 - 2,9 (m,1H), 1,32 (d,6H) ppm.

Beispiel 2

Elektrosynthese von 4-(Dimethoximethyl)phenylpivalat

In der in Beispiel 1 beschriebenen Zelle wurde die Elektrooxidation mit einem Elektrolyten der folgenden Zusammensetzung durchgeführt:
57 g (5,0 Gew.-%) 4-Methylphenylpivalat
30,6 g (2,7 Gew.-%) Pivalinsäure
3,4 g (0,3 Gew.-%) Kaliumbenzosulfonat
1050 g (92 Gew.-%) Methanol

| | |
|---|---|
| Temperatur: | 40 °C |
| Stromdichte: | 1 A/dm$^2$ |
| Durchfluß: | 190 l/h |

Elektrolyse mit 9,5 F/Mol 4-Methylphenylpivalat
Es wurde analog Beispiel 1 aufgearbeitet und durch Destillation bei 132 °C (0,3 mbar) 49,5 g (66 %) 4-(Dimethoximethyl)phenylpivalat erhalten.
MS (CI): m/e = 252
$^1$H-NMR ($CDCl_3$, 300 MHz): = δ 7,40 - 7,50 (2H), 7,0 - 7,10 (2H), 5,40 (s,1H) 3,32 (s,6H), 1,38 (s,9H) ppm.

Beispiel 3

Elektrosynthese von 4-(Dimethoximethyl)phenylpivalat

In der in Beispiel 1 beschriebenen Zelle wurde die Elektrooxidation mit einem Elektrolyten der folgenden Zusammensetzung durchgeführt:
57 g (5,05 Gew.-%) 4-Methylphenylpivalat
18 g (1,6 Gew.-%) Essigsäure
3,4 (0,3 Gew.-%) Kaliumbenzosulfonat
1050 g (93,05 Gew.-%) Methanol

| | |
|---|---|
| Temperatur: | 40 °C |
| Stromdichte: | 1 A/dm$^2$ |
| Durchfluß: | 190 l/h |

Elektrolyse mit 10 F/Mol 4-Methylphenylpivalat

Es wurde analog Beispiel 1 aufgearbeitet und durch fraktionierte Destillation 41,8 g (55 %) 4-(Dimethoximethyl)phenylpivalat erhalten

Beispiel 4

Elektrosynthese von 4-(Dimethoximethyl)phenylpivalat

In der in Beispiel 1 beschriebenen Zelle wurde die Elektrooxidation mit einem Elektrolyten der folgenden Zusammensetzung durchgeführt:
53 g (5,0 Gew.-%) 4-Methylphenylpivalat
3,2 g (0,3 Gew.-%) Kaliumbenzosulfonat
1000 g (94,7 Gew.-%) Methanol

| | |
|---|---|
| Temperaur: | 40 °C |
| Stromdichte: | 1 A/dm$^2$ |
| Durchfluß: | 190 l/h |

Elektrolyse mit 15,5 F/Mol 4-Methylphenylpivalat

Es wurde analog Beispiel 1 aufgearbeitet. Dabei wurden 31,7 g (46 %) 4-(Dimethoximethyl)phenylpivalat erhalten.

Beispiel 5

Elektrosynthese von 4-(Dimethoximethyl)phenylpivalat

In der in Beispiel 1 beschriebenen Zelle wurde die Elektrooxidation mit einem Elektrolyten der folgenden Zusammensetzung durchgeführt:
115 g (10,0 Gew.-%) 4-Methylphenylpivalat
36 g (3,1 Gew.-%) Essigsäure
3,5 g (0,3 Gew.-%) Kaliumbenzosulfonat
1000 g (86,6 Gew.-%) Methanol

| | |
|---|---|
| Temperatur: | 40 °C |
| Stromdichte: | 3 $A/dm^2$ |
| Durchfluß: | 190 l/h |

Elektrolyse mit 7,5 F/Mol 4-Methylphenylpivalat

Es wurde analog Beispiel 1 aufgearbeitet. Dabei wurden nach fraktionierter Destillation 84,7 g (56 %) 4-(Dimethoximethyl)phenylpivalat erhalten.

Beispiel 6

Elektrosynthese von Methyl-4-(dimethoximethyl)phenylcarbonat

In der in Beispiel 1 beschriebenen Zelle wurde die Elektrooxidation mit einem Elektrolyten der folgenden Zusammensetzung durchgeführt:
55,4 g (4,9 Gew.-%) Methyl-4-methylphenylcarbonat
20 g (1,8 Gew.-%) Essigsäure
3,4 g (0,3 Gew.-%) Kaliumbenzosulfonat
1050 g (93,0 Gew.-%) Methanol

| | |
|---|---|
| Temperatur: | 40 °C |
| Stromdichte: | 1 $A/dm^2$ |
| Durchfluß: | 190 l/h |

Elektrolyse mit 9,5 F/Mol Methyl-4-methylphenylcarbonat

Aufarbeitung: Nach Beendigung der Elektrolyse wurde der Austrag in einem Rotationsverdampfer eingeengt und der Rückstand durch fraktionierte Destillation gereinigt. Man erhielt bei 115 - 120 °C (0,2 mbar) 46,1 g (61 %) Methyl-4-(dimethoximethyl)phenylcarbonat.
MS (Cl): m/e = 226
$^1$H-NMR ($CDCl_3$, 300 MHz): $\delta$ = 7,4 - 7,5 (2H), 7,15 - 7,2 (2H), 5,40 (S,1H), 3,92 (s,3H), 3.32 (s,6H) ppm.

Beispiel 7

Elektrosynthese von Methyl-4-(dimethoximethyl)phenylcarbonat

In der in Beispiel 1 beschriebenen Zelle wurde die Elektrooxidation mit einem Elektrolyten der folgenden Zusammensetzung durchgeführt:
116 g (10,0 Gew.-%) Methyl-4-methylphenylcarbonat
42 g (3,6 Gew.-%) Essigsäure
3,5 g (0,3 Gew.-%) Kaliumbenzosulfonat
1000 g (86,1 Gew.-%) Methanol

| | |
|---|---|
| Temperatur: | 40 °C |
| Stromdichte: | 3 $A/dm^2$ |
| Durchfluß: | 190 l/h |

Elektrolyse mit 7,55 F/Mol Methyl-4-methylphenylcarbonat

Es wurde analog Beispiel 6 aufgearbeitet und durch fraktionierte Destillation 95,8 g (61 %) Methyl-4-(dimethoximethyl)phenylcarbonat erhalten.

Beispiel 8 (Vergleichsbeispiel)

Elektrosynthese von 4-Acetoxybenzaldehyddimethylacetal

In der in Beispiel 1 beschriebenen Zelle wurde die Elektrooxidation mit einem Elektrolyten der folgenden Zusammensetzung durchgeführt:
57,4 g (5,0 Gew.-%) 4-Acetoxytoluol
139 g (12,1 Gew.-%) Essigsäure
7,3 g (0,6 Gew.-%) Natriumtetrafluoroborat
945 g (82,3 Gew.-%) Methanol

| | |
|---|---|
| Temperatur: | 40 °C |
| Stromdichte: | 1,6 A/$dm^2$ |
| Durchfluß: | 190 l/h |

Elektrolyse mit 27 F/Mol 4-Acetoxytoluol

Aufarbeitung: Nach Beendigung der Elektrolyse wurde der Austrag mit Natriumcarbonat neutralisiert, filtriert und das Filtrat im Rotationsverdampfer eingedampft. Der Rückstand wurde in Methyl-tert.-butylether aufgenommen und filtriert. Nach Destillation des Lösungsmittels erhielt man durch fraktionierte Destillation bei 118 °C (0,5 mbar) 14,8 g (18 %) 4-Acetoxybenzaldehyddimethylacetal.
MS (CI): m/e = 210
$^1$H-NMR ($CDCl_3$, 250 MHz): $\delta$ = 7,40 - 7,50 (2H), 7,0 - 7,1 (2H), 5,40 (s,1H), 3,32 (s,6H), 2,30 (s,3H) ppm.

Beispiel 9

Herstellung von 4-Formylphenylisobutyrat

30 g 4-(Dimethoximethyl)phenylisobutyrat wurden mit 90 g Salzsäure (1 M) eine Stunde bei Raumtemperatur gerührt. Die wäßrige Phase wurde abgetrennt und dreimal mit Methyl-tert.-butylether extrahiert. Die vereinigten organischen Phasen wurden getrocknet und im Rotationsverdampfer eingedampft. Der Rückstand wurde fraktioniert. Man erhielt bei 105 °C (0,1 mbar) 18 g (74 %) 4-Formylphenylisobutyrat.
$^1$H-NMR ($CDCl_3$, 300 MHz): $\delta$ = 9,98 (s,1H), 7,90 - 8,0 (2H), 7,2 -7,3 (2H), 2,83 (m,1H), 1,34 (d,6H) ppm.

Beispiel 10

Herstellung von 4-Formylphenylpivalat

60 g 4-(Dimethoximethyl)phenylpivalat wurden eine Stunde mit 186 g Salzsäure (1 M) bei Raumtemperatur gerührt. Die wäßrige Phase wurde abgetrennt und dreimal mit Methyl-tert.-butylether extrahiert. Die vereinigten organischen Phasen wurden getrocknet und im Rotationsverdampfer eingedampft. Der Rückstand wurde fraktioniert. Man erhielt bei 102 °C (0,1 mbar) 43,6 g (90 %) 4-Formylphenylpivalat.
$^1$H-NMR ($CDCl_3$, 300 MHz): $\delta$ = 9,98 (2,1H), 7,90 - 7,95 (2H), 7,2 -7,25 (2H), 1,38 (s,9H) ppm.

Beispiel 11

Herstellung von Methyl-4-formylcarbonat

40 g Methyl-4-(dimethoximethyl)phenylcarbonat wurden eine Stunde mit 120 g Salzsäure (1 M) bei Raumtemperatur gerührt. Die wäßrige Phase wurde abgetrennt und dreimal mit Methyl-tert.-butylether extrahiert. Die vereinigten organischen Phasen wurden getrocknet und in einem Rotationsverdampfer eingedampft. Der Rückstand wurde fraktioniert. Man erhielt bei 92 °C (0,1 mbar) 25,8 g (81 %) Methyl-4-formylphenylcarbonat.
$^1$H-NMR ($CDCl_3$, 300 MHz): $\delta$ = 10,0 (s,1H), 7,90 - 7,96 (2H), 7,15 - 7,22 (2H), 3,94 (s,3H) ppm.

## Patentansprüche

1. Benzaldehydderivate der allgemeinen Formel

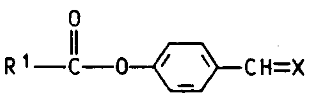

I,

in der X ein Sauerstoffatom oder zwei $R^2O$-Gruppen, in denen $R^2$ für eine Methyl- oder Ethylgruppe steht, bedeutet, $R^1$ für eine iso-Propyl-oder tert.-Butylgruppe steht und $R^1$ für den Fall, daß X für die beiden $R^2O$-Gruppen steht, auch eine Alkoxigruppe mit 1 bis 4 C-Atomen bedeuten kann

2. Benzaldehyddialkylacetale der allgmeinen Formel

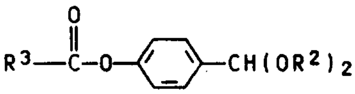

II,

in der $R^2$ die in Anspruch 1 genannte Bedeutung hat und $R^3$ eine iso-Propyl- oder tert.-Butylgruppe oder eine Alkoxigruppe mit 1 bis 4 C-Atomen bedeutet.

3. Benzaldehyde der allgmeinen Formel

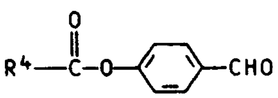

III,

in der $R^4$ eine iso-Propyl- oder tert.-Butylgruppe bedeutet.

4. 4-(Dimethoximethyl)phenylisobutyrat.

5. 4-(Dimethoximethyl)phenylpivalat.

6. Methyl-4-(dimethoximethyl)phenylcarbonat.

7. 4-Formylphenylisobutyrat.

8. 4-Formylphenylpivalat.

9. Verfahren zur Herstellung der Benzaldehyddialkylacetale nach Anspruch 2, dadurch gekennzeichnet, daß man Toluole der allgemeinen Formel

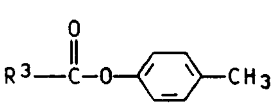

IV,

in Gegenwart eines Alkanols der Formel $R^2OH$ elektrochemisch oxidiert, wobei $R^2$ und $R^3$ die in Anspruch 2 genannte Bedeutung haben.

10. Verwendung der Benzaldehydderivate gemäß Anspruch 1 als Riechstoffe oder Riechstoffvorprodukte.

## Claims

1. A benzaldehyde derivative of the formula

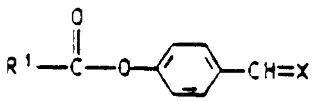

I

where X is oxygen or two $R^2O$ groups where $R^2$ is methyl or ethyl, $R^1$ is isopropyl or tert-butyl, and $R^1$ can also be alkoxy of 1 to 4 carbons when X is two $R^2O$ groups.

**2.** A benzaldehyde dialkyl acetal of the formula

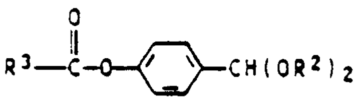

II

where $R^2$ has the meanings specified in claim 1, and $R^3$ is isopropyl or tert-butyl or alkoxy of 1 to 4 carbons.

**3.** A benzaldehyde of the formula

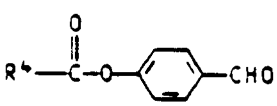

III

where $R^4$ is isopropyl or tert-butyl.

**4.** 4-(Dimethoxymethyl)phenyl isobutyrate.

**5.** 4-(Dimethoxymethyl)phenyl pivalate.

**6.** Methyl 4-(dimethoxymethyl)phenyl carbonate.

**7.** 4-Formylphenyl isobutyrate.

**8.** 4-Formylphenyl pivalate.

**9.** A process for preparing a benzaldehyde dialkyl acetal as claimed in claim 2, which comprises electrochemical oxidation of a toluene of the formula

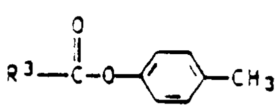

IV

in the presence of an alkanol of the formula $R^2OH$ where $R^2$ and $R^3$ have the meanings specified in claim 2.

**10.** The use of a benzaldehyde derivative as claimed in claim 1 as fragrance or precursor thereof.

**Revendications**

**1.** Dérivés de benzaldéhyde de formule générale

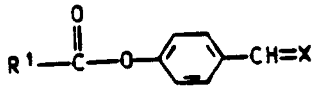

I.

où X représente un atome d'oxygène ou deux groupements $R^2O$ dans lesquels $R^2$ est mis pour un groupement méthyle ou éthyle, $R^1$ est mis pour un groupement isopropyle ou tert.-butyle et, dans le cas où X est mis pour les deux groupements $R^2O$, $R^1$ peut aussi représenter un groupement alcoxy à 1-4 atomes de carbone.

**2.** Benzaldéhydedialkylacétals de formule générale

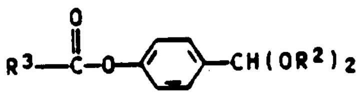

II.

dans laquelle $R^2$ a la signification donnée dans la revendication 1 et $R^3$ représente un groupement isopropyle ou tert.-butyle ou un groupement alcoxy à 1-4 atomes de carbone.

**3.** Benzaldéhydes de formule générale

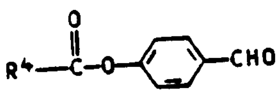

III.

dans laquelle $R^4$ représente un groupement isopropyle ou tert.-butyle.

**4.** Isobutyrate de 4-(dimethoxyméthyl)phényle.

**5.** Pivalate de 4-(diméthoxyméthyl)phényle.

**6.** Carbonate de méthyle et de 4-(diméthoxyméthyl)-phényle.

**7.** Isobutyrate de 4-formylphényle.

**8.** Pivalate de 4-formylphényle.

**9.** Procédé de préparation des benzaldéhydedialkylacétals selon la revendication 2, caractérisé en ce qu'on oxyde électrochimiquement des toluènes de formule générale

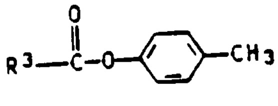

IV.

en présence d'un alcanol de formule $R^2OH$, $R^2$ et $R^3$ ayant la signification donnée dans la revendication 2.

**10.** Utilisation des dérivés de benzaldéhyde selon la revendication 1 comme matières odorantes ou comme progéniteurs de matières odorantes.